# EUROPÄISCHE PATENTSCHRIFT

(11) **EP 2 554 133 B1**
(45) Veröffentlichungstag und Bekanntmachung des Hinweises auf die Patenterteilung: **22.01.2014**
(21) Anmeldenummer: 11176289.4
(22) Anmeldetag: 02.08.2011
(51) Int. Cl.: A61B 18/14

(54) **Instrument zur Gefäßfusion und -trennung mit zwei Branchen und mit einer gekrümmten Elektrode**
Instrument for vessel fusion and separation with two arms and with a curved electrode
Instrument pour la fusion et la séparation de vaisseaux avec deux branches et avec une électrode courbe

(43) Veröffentlichungstag der Anmeldung: 06.02.2013
(73) Patentinhaber: ERBE Elektromedizin GmbH, 72072 Tübingen (DE)
(72) Erfinder: Hiller, Jürgen, 72581 Dettingen (DE); Kühner, Ralf, 70567 Stuttgart (DE); Baur, Thomas, 72108 Rottenburg (DE); Rydzewski, Viktoria, 72622 Nürtingen (DE)
(74) Vertreter: Rüger, Barthelt & Abel

(56) Entgegenhaltungen:
- US-A- 6 113 598
- US-A1- 2002 115 997
- US-A1- 2007 078 456
- US-A1- 2011 082 494

## Beschreibung

Die Erfindung betrifft ein Instrument zum Abklemmen, Verschließen und Durchtrennen von Gewebe bzw. Gefäßen, insbesondere Blutgefäße am lebenden Körper eines menschlichen oder tierischen Patienten.

Prinzipiell sind Instrumente zum Verschluss und zur Trennung von Blutgefäßen bekannt. Ein Beispiel ist der DE 602 26 015 T2 zu entnehmen. Ein weiteres Beispiel, das die Merkmale des Oberbegriffs des Anspruchs 1 offenbart, geht aus der US 2002/0115997 A1 hervor.

Solche Instrumente umfassen einen länglichen Schaft, an dessen distalen Ende ein Werkzeug mit zwei Branchen zum Abklemmen eines Blutgefäßes angeordnet ist. Außerdem ist dort ein längsverschiebbares Messer angeordnet, um ein gefasstes und koaguliertes Gefäß durchtrennen zu können. An dem proximalen Ende des Schafts ist ein Handgriff mit Betätigungsmitteln vorgesehen, über die die Branchen geschlossen und das Messer betätigt werden können. Die Branchen sind als Elektroden ausgebildet, um das zwischen ihnen geklemmte Gefäß erwärmen und die Gefäßwände durch Koagulation zur Fusion bringen zu können.

Bei der Durchtrennung von Blutgefäßen muss sicher gestellt werden, dass die Enden des durchtrennten Gefäßes verlässlich geschlossen bleiben. Dabei müssen weitere Bedingungen erfüllt werden. Zum Beispiel soll die Schädigung umliegenden Gewebes vermieden werden. Der Gefäßverschluss soll weitgehend unabhängig von der Größe der Gefäße und der Dicke der Gefäßwände erreichbar sein. Dabei muss im Weiteren auf die beengten Platzverhältnisse Rücksicht genommen werden, die spätestens dann zu beachten sind, wenn das Instrument endoskopisch oder laproskopisch eingesetzt werden soll.

Davon ausgehend ist es Aufgabe der Erfindung, ein Instrument zur Gefäßfusion und -trennung zu schaffen, mit dem sich Gefäße in hoher Qualität verschließen und trennen lassen.

Diese Aufgabe wird mit dem Instrument nach Anspruch 1 gelöst.

Das erfindungsgemäße Instrument dient dem Verschließen und Durchtrennen von Blutgefäßen. Es weist zwei Branchen auf, von denen wenigstens eine in Bezug auf die andere beweglich, vorzugsweise schwenkbar, gelagert ist. Es können beide Branchen bezüglich des Schafts des Instruments beweglich gelagert sein. Es ist auch möglich, dass die erste der Branchen starr mit dem Schaft verbunden ist während die andere beweglich gelagert ist. Letzteres wird gegenwärtig bevorzugt. Die Lagerung der Branchen kann so ausgeführt sein, dass nicht uniformes Gewebe leicht gefasst werden kann.

Die erste Branche weist einen elektrisch isolierenden bspw. aus Keramik oder auch einen geeigneten Kunststoff bestehenden Grundkörper mit einer rinnenartigen Ausnehmung für eine Elektrode und einem Führungsschlitz für ein Messer auf. Die rinnenartige Ausnehmung ist der Form der Elektrode angepasst. Die Elektrode weist eine konkave Ausnehmung auf. Sie ist bspw. als Zylinderschale ausgebildet. Sie kann jedoch auch einen U-Querschnitt mit geraden Schenkeln aufweisen. Vorzugsweise wird sie durch ein dünnwandiges Blechteil gebildet, das durch die Abstützung in dem Grundkörper die nötige mechanische Stabilität erhält.

Die Elektrode ist über ein elektrisches Anschlussmittel mit einer sich durch den Schaft erstreckenden Leitung verbunden. Das Anschlussmittel ist ein vorzugsweise isolierter elektrischer Leiter. Er führt durch geeignete Kanäle oder Durchgänge des Grundkörpers und kann z.B., insbesondere in der Nähe eines Gelenks, bei dem die zweite Branche beweglich an der ersten Branche gelagert ist, an der Außenseite des Grundkörpers verlaufen. Der Grundkörper kann dazu mit einer längs verlaufenden Ausnehmung versehen sein, in der die Leitung angeordnet ist. Dadurch kann sichergestellt werden, dass der Kopf des Instruments einen maximalen Außenquerschnitt nicht übersteigt und das Instrument z.B. zum laproskopischen Einsatz durch einen Trokar geschoben werden kann.

Vorzugsweise wird als elektrischer Leiter ein physiologisch verträgliches Material wie Silber, Titan oder dergleichen genutzt. Der Leiter kann als massiver Runddraht, Hohldraht, als Flachdraht, als Litze, als Band oder dergleichen ausgeführt sein.

Das Messer ist zumindest in dem Führungsschlitz des Grundkörpers präzise geführt. Vorzugsweise ist das Messer dabei mittig zu der Elektrode und auch mittig zu der zweiten Branche geführt, in deren Längsschlitz sie einfahren kann. Die Führung des Messers kann beim Vorschieben zumindest teilweise von dem Längsschlitz der zweiten Branche geleistet werden.

Durch die präzise Führung des Messers kann sichergestellt werden, dass der Schnitt beim Durchtrennen eines Gefäßes mittig in dem zuvor fusionierten Bereich des Gefäßes gesetzt wird. Damit haben beide Enden des schlussendlich durchtrennten Gefäßes gleich lange fusionierte Abschnitte. Die Gefahr, dass es wegen eines nicht präzise mittig gesetzten Schnittes zu unkontrollierten Blutungen kommt, ist minimiert.

Vorzugsweise umfasst das Messer eine Führungsleiste und eine Klinge, die fest miteinander verbunden sind. Die Führungsleiste ist vorzugsweise ein längliches sich in Bewegungsrichtung des Messers erstreckendes Element zum Beispiel in Form einer Kunststoffschiene. Sie kann mit einer Klinge aus Metall verbunden sein. Die Klinge kann jedoch auch aus Keramik, Diamant, Kunststoff oder sonstigem Material bestehen. Die Führungsleiste kann alternativ aus einem Metall bestehen. Es ist auch möglich, die Führungsleiste durch eine an der Klinge ausgebildete Verdickung zu bilden, so dass Führungsleiste und Klinge nahtlos einteilig miteinander aufgebaut sind. Der zweiteilige Aufbau wird aber bevorzugt.

Das seitliche Spiel des Messers in dem Führungsschlitz und/oder in dem Längsschlitz ist vorzugsweise geringer als das Fünffache der Dicke D der Klinge. Vorzugsweise ist das seitliche Spiel des Messers höchstens etwa so groß wie die Dicke der Klinge. Dadurch wird eine präzise mittige Führung des Messers und somit eine mittige Schnittführung im fusionierten Gewebebereich erzielt. Die seitliche Führung des Messers kann durch den Führungsschlitz erbracht werden. Die seitliche Führung des Messers kann alternativ durch den Längsschlitz erbracht werden. Vorzugsweise wird die seitliche Führung sowohl durch den Führungsschlitz als auch durch den Längsschlitz erbracht.

Unter seitlichem Spiel des Messers wird der Abstand der Flanken der Führungsleiste von der Flanke des Führungsschlitzes oder Längsschlitzes bei mittiger Messerlage verstanden. Die seitliche Beweglichkeit des Messers entspricht somit dem doppelten Spiel.

Vorzugsweise weist das Messer eine gerade, der Elektrode zugewandte und an dieser anliegende längs verlaufende Gleitkante auf. Die Gleitkante kann relativ kurz ausgebildet sein und schließt sich vorzugsweise unmittelbar an die Schneidkante des Messers an. Somit wird sichergestellt, dass das zwischen den Branchen gefasste Gefäß auf ganzem Querschnitt durchtrennt wird und kein Geweberest undurchtrennt bleibt.

Die Schneidkante ist an dem Messer vorzugsweise stirnseitig angeordnet. Sie steht vorzugsweise unter einem Winkel zu der Bewegungsrichtung des Messers. Dieser Winkel kann ein spitzer Winkel sein. Vorzugsweise ist die Schneidkante gerade ausgebildet, wobei sie auf die Führungsleiste hin läuft. Vorzugsweise überragt die Führungsleiste die Schneidkante, um zu verhindern, dass Teile des zu durchtrennenden Gefäßes über das Messer gedrängt und somit vom Schnitt nicht erfasst werden.

Die zweite Branche weist, zumindest an ihrer der Elektrode zugewandten Seite, eine elektrisch leitende Fläche auf. Ist die zweite Branche aus einem nicht metallischen Material kann diese Fläche durch ein dünnes Elektrodenblech gebildet sein. Vorzugsweise aber ist die zweite Branche massiv aus Metall ausgebildet. Unabhängig davon weist die zweite Branche an ihrer der Elektrode zugewandten Seite eine der Form der Elektrode folgende Form auf. Mit anderen Worten, zwischen der Elektrode und der zweiten Branche wird ein Abstandsspalt von im Wesentlichen konstanter oder zur Mitte hin abnehmender Spaltweite gebildet. Unabhängig davon kann die Oberfläche der zweiten Branche profiliert, bspw. gerippt, genoppt oder anderweitig geformt sein. Auch die Elektrode kann mit einer Oberflächenprofilierung versehen sein. Auch wenn die Profilierungen der Elektrode und der zweiten Branche gegebenenfalls unterschiedlich sind, haben sie vorzugsweise zueinander passende einander folgende Grundformen zur Festlegung eines Abstandsspalts mit vorzugsweise zur Mitte hin abnehmender Spaltweite.

Die Haltemittel zur Fixierung der Elektrode an der ersten Branche sind an den oberen, einander gegenüber liegenden Rändern der Ausnehmung ausgebildet. Erfindungsgemäß sind diese Haltemittel durch elektrisch isolierende Vorsprünge gebildet, die bspw. aus dem Material des Grundkörpers bestehen. Sie ragen nach inner in Richtung auf die Ausnehmung zu und über-den oberen Rand der Elektrode. Vorzugsweise legen die Vorsprünge mit der zweiten Branche bei geschlossenem Instrument einen Spalt mit einem Abstand B fest, der höchstens so groß ist, wie die Weite A des Abstandsspalts in Nachbarschaft zu den Haltemitteln.

Zwischen der zweiten Branche und solchen Vorsprüngen kann Gewebe des abzuklemmenden Gefäßes gefasst werden, das wegen der elektrisch isolierenden Eigenschaften der Vorsprünge hier nicht koaguliert wird. Das Fassen des Gewebes ist auch dann sichergestellt, wenn die Branchen aufgrund von Gewebeeigenschaften nicht symmetrisch schließen. Wird beispielesweise ein Gefäß gefasst, dessen Querschnitt sich über die Länge verändert, kann dies dazu führen, dass die seitlichen Abstände B unterschiedliche Werte aufweisen können d.h. nicht gleich groß sind. Trotzdem wird das Gefäß sicher gehalten. Ein Verrutschen des Gefäßes ist auch dann sicher ausgeschlossen, wenn das zwischen der Branche und der Elektrode gefasste Gewebe durch Wärmeeinwirkung und Koagulation schrumpft und dort die Klemmung abnimmt. Ein seitliches Verrutschen des Gefäßes und infolgedessen ein späteres Durchtrennen des Gefäßes an ungeeigneter Stelle bei nachfolgender Aktivierung des Messers wird auf diese Weise sicher ausgeschlossen.

Die durch die Vorsprünge gebildeten Haltemittel haben eine Doppelfunktion: sie halten die Elektrode und sie Verbessern das Halten des Gefäßes.

Zwischen der ersten und der zweiten Branche ist vorzugsweise ein Abstandhaltemittel vorgesehen. Dieses Abstandshaltemittel verhindert, dass die zweite Branche die Elektrode berührt und es somit zum Kurzschluss kommt. Außerdem verhindert das Abstandhaltemittel, dass unbeabsichtigt ein zu großer Druck auf das Gefäß ausgeübt und dieses somit abgequetscht wird. Vorzugsweise ist das Abstandshaltemittel durch einen metallischen Vorsprung gebildet, dem ein nicht metallischer Anschlag auf der Gegenseite zugeordnet ist. Der metallische Vorsprung ist vorzugsweise elektrisch beaufschlagt, d.h. mit der Elektrode oder mit der zweiten Branche verbunden. Dadurch werden Fehler vermieden, die auftreten könnten, wenn biologisches Gewebe zwischen den Vorsprung und die ihm zugeordnete Anlagefläche gelangt. Durch die elektrische Bestromung kann, insbesondere wenn der Vorsprung in der Nähe der Gegenpolelektrode angeordnet ist, ein gewisser Stromfluss in das gefasste biologische Gewebe eingeleitet werden, so dass es schrumpft und die Einstellung des gewünschten Minimalabstands zwischen der zweiten Branche und der Elektrode nicht mehr behindert. Bei einem alternativen Ausführungsbeispiel ist es möglich, dass das Abstandshaltemittel aus einem elektrisch nicht leitenden Material gebildet ist. Dadurch kann die Kurzschlussgefahr zwischen den Branchen reduziert werden. Zusätzlich ermöglicht dies, den Koagulationsbereich zu erweitern.

Weitere Einzelheiten vorteilhafter Ausführungsformen der Erfindung ergeben sich aus der nachfolgenden Beschreibung, Unteransprüchen oder der Zeichnungen. Es zeigen:
Figur 1 ein erfindungsgemäßes Instrument in schematisierter Darstellung.
Figur 2 die Branchen und das Messer des Instruments, in schematisierter Seitenansicht.
Figur 3 die Branchen und das Messer gemäß Figur 2, in teilweise aufgeschnittener Prinzipansicht.
Figur 4 die Branchen und das Messer, in Vertikalschnittdarstellung mit Blick in Richtung der Längsachse, in vereinfachter, schematisierter Darstellung bei geschlossenem Werkzeug,
Figur 5 den Kopf des erfindungsgemäßen Instruments, in perspektivischer Ansicht und
Figur 6 den Grundkörper und das Messer, in Vertikalschnittdarstellung mit Blick in Richtung der Längsachse, in vereinfachter, schematisierter Darstellung.

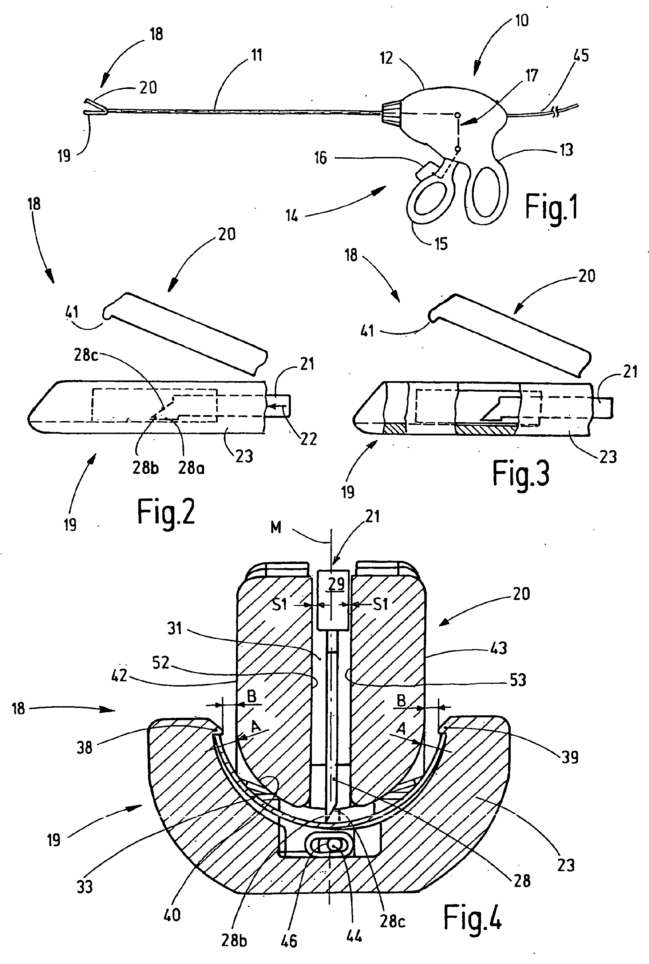

In Figur 1 ist ein Instrument 10 veranschaulicht, das zum Abklemmen, Fusionieren und Durchtrennen eines Gefäßes z.B. eine Blutgefäßes beim Operieren an einem menschlichen oder tierischen Körper dienen kann. Das Instrument 10 dient insbesondere der endoskopischen Chirurgie. Es umfasst einen schlanken Schaft 11, der an seinem proximalen Ende an einem Gehäuse 12 gehalten ist. Dieses weist Handhabungsmittel beispielsweise einen Handgriff 13, sowie Betätigungselemente 14 auf. Diese können z.B. aus einem schwenkbaren Griffteil 15 und/oder einem Betätigungsknopf- oder -hebel 16 sowie gegebenenfalls weiteren Elementen, z.B. elektrischen Schaltern bestehen. Die Betätigungselemente 14 sind über in Figur 1 lediglich schematisch gestrichelt angedeutete Kraftübertragungsmittel 17 mit einem an dem distalen Ende des Schafts 11 angeordneten Anwendungsteil 18 verbunden. Dieses Anwendungsteil oder Kopf 18 des chirurgischen Instruments - umfasst mindestens die in den Figuren 2 und 3 veranschaulichten Elemente nämlich eine erste Branche 19, eine zweite Branche 20 und ein Messer 21. Die Branchen 19, 20 dienen zum Abklemmen eines Gefäßes, indem sie aufeinander zu bewegt werden und das Gefäß zwischen einander festklemmen. Das Messer 21 dient dazu, das Gefäß zu durchtrennen.

Im vorliegenden Ausführungsbeispiel ist die Branche 19 starr mit dem Schaft 11 verbunden, während die Branche 20 durch Betätigung des Griffteils 15 auf die Branche 19 hin oder von dieser weg zu schwenken ist. Hingegen wird das Messer 21 durch Betätigung des Betätigungsknopfes 16 in Längsrichtung des Schafts 11, wie in Figur 2 durch einen Pfeil 22 angedeutet, vorwärts geschoben.

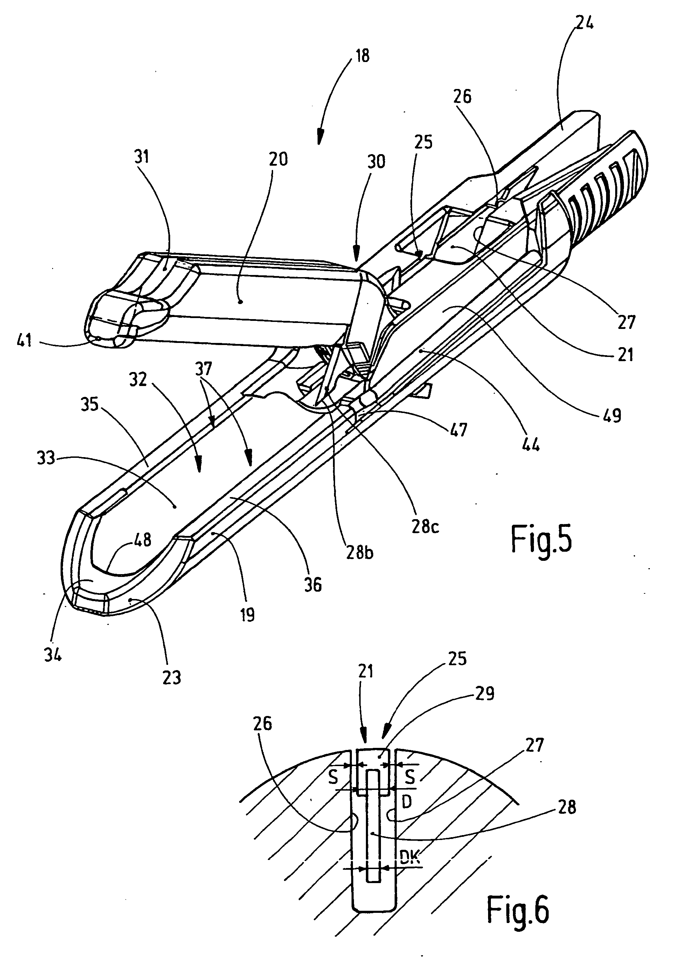

Die Branche 19 ist mehrteilig aufgebaut. Sie umfasst einen z.B. aus Kunststoff oder Keramik bestehenden Grundkörper 23, wie er insbesondere aus Figur 4 ersichtlich ist. Der Grundkörper 23 ist an seinem schaftseitigen Ende mit dem Schaft 11 starr verbunden, wozu er einen aus Figur 5 ersichtlichen Ansatz 24 aufweist. Von diesem ausgehend erstreckt sich der längliche Grundkörper 23 weg, wobei er zunächst einen Führungsschlitz 25 für das Messer 21 aufweist. Der sich in Längsrichtung durch den länglichen Rundkörper 23 erstreckende Führungsschlitz 25 weist zwei Schlitzflanken 26, 27 auf, die einander parallel im Abstand gegenüber liegen und zwischen einander das Messer 21 führen. Dies ist insbesondere in Figur 6 veranschaulicht. Das Messer 21 hat dabei in Mittellage zu jeder Schlitzflanke 26, 27 einen als Spiel S bezeichneten Abstand, der jeweils vorzugsweise höchstens so groß ist, wie die Dicke D des Messers 21.

Letzteres besteht aus einer Klinge 28, die an ihrer oberen Kante mit einer Führungsleiste 29 versehen ist. D ist dabei als die Dicke der Führungsleiste 21 definiert (Figur 6). Als Dicke DK ist die Dicke der Klinge 28 definiert. Vorzugsweise ist das Spiel S höchstens so groß wie das Fünffache der Dicke DK. Im besseren Fall ist die Dicke DK höchstens so groß wie das Doppelte des Spiels S. Mit anderen Worten, das Messer 21 kann sich um maximal ein Maß von zweimal S zwischen den Flanken 26, 27 hin und her bewegen. Im Idealfall ist die Dicke DK höchstens so groß wie das Spiel S.

Das Messer 21 weist vorzugsweise eine z.B. an der Klinge 28 angeordnete Gleitkante 28a auf, die an dem Boden der Branche 19 gleiten kann und das Messer 21 in der Höhe führt (Figur 2). Dadurch kann die sich unten an die Schneidkante 28c anschließende Spitze 28b der Klinge 28 (Figur 4 und 5) an der Elektrode 33 oder mit ganz geringem Abstand zu dieser laufen, ohne sich zu verschleißen.

In gerader Verlängerung an den Führungsschlitz 25 schließt sich ein Lager- oder Scharnierbereich 30 an, in dem die zweite Branche 20 schwenkbar an dem Grundkörper 23 gelagert ist. Die Branche 20 ist mit dem in Figur 5 nicht weiter veranschaulichten Kraftübertragungsmittel 17 verbunden, um durch dieses um eine quer zu dem Grundkörper 23 stehender Achse geschwenkt zu werden.

Der Führungsschlitz 25 setzt sich als Längsschlitz 31 durch die Branche 20 fort. Der Längsschlitz 31 ist, zumindest nach unten, d.h. nach unten zu dem Grundkörper 23 hin offen. Vorzugsweise stimmt seine Breite wenigstens näherungsweise mit der Breite des Führungsschlitzes 25 überein. Die Schlitzflanken 52, 53 des Längsschlitzes 31 sind ebene, parallel zueinander orientierte Flächen. Mit dem Messer 21 definieren sie die Spielmaße S1 für die das oben zu dem Spiel S gesagte entsprechend gilt.

Der Grundkörper 23 weist unterhalb der Branche 20 eine rinnen- oder muldenförmige Ausnehmung 32 auf, in der eine Elektrode 33 angeordnet ist. Die Elektrode 33 wird im vorliegenden Ausführungsbeispiel durch ein dünnes zylinderschalenförmiges Blech gebildet. Sie weist an der der Branche 19 zugewandten Seite eine gekrümmte, vorzugsweise konkave, vorzugsweise geschlossene, glatte Fläche auf. Die Elektrode 33 erstreckt sich dabei über nahezu die gesamte Länge der Ausnehmung 32, wobei sie jedoch an dem vorderen Ende des Grundkörpers 23 einen Bereich 34 frei lässt. An den oberen Rändern 35, 36 des Grundkörpers 23 sind Haltemittel 37 für die Elektrode 33 angeordnet. Diese sind, wie Figur 4 zeigt, durch Rastvorsprünge 38, 39 gebildet, die aus elektrisch isolierendem Material bestehen und vorzugsweise einstückiger Bestandteil des Grundkörpers 23 sind. Die Rastvorsprünge 38, 39 bilden Haltenasen, die den oberen Rand der Elektrode 33 übergreifen. Ihre Länge übersteigt etwas die Dicke der Elektrode 33, so dass sie deren freiliegende Elektrodenfläche etwas überragen.

Die obere Branche 20 weist an ihrer der Elektrode 33 zugewandten Unterseite ein der Form der Elektrode 33 folgende Elektrodenfläche 40 auf. Ist die Elektrode 33 bspw. zylinderschalenförmig ausgebildet, folgt die Elektrodenfläche 40 ebenfalls einem Kreisbogen, wobei dieser einen etwas geringeren Radius aufweist. Unabhängig von dieser Grundform können sowohl die Elektrode 33 als auch die Elektrodenfläche 40 lokale Formabweichungen in Form von Ausnehmungen, Vorsprüngen, Noppen, Rippen oder dergleichen aufweisen.

An dem distalen Ende der zweiten Branche 20 ist ein sich zu dem Grundkörper 23 hin erstreckender nasenartiger Vorsprung 41 vorgesehen, der sich bei geschlossenem Instrument an dem endständigen Bereich 34 des Grundkörpers 23 abstützt. In diesem Zustand ist zwischen der Elektrodenfläche 40 und der Innenseite der Elektrode 33 ein Abstandsspalt mit der Weite A festgelegt. Vorzugsweise ist diese Weite A, zumindest geringfügig, größer als der Abstand B zwischen dem Vorsprung 38 bzw. 39 und der Elektrodenfläche 40 oder der sich daran anschließenden Flanke 42, 43 der Branche 20.

Die Elektrode 33 ist, wie aus den Figuren 4, 5 ersichtlich, über einen elektrischen Leiter 44 an eine elektrische Quelle angeschlossen. Der Leiter 44 führt dabei durch den Schaft 11 zunächst in das Gehäuse 12 dort zu einem nicht weiter veranschaulichten Schalter und gegebenenfalls über eine Zuleitung 45 zu einem versorgenden Gerät.

Ebenso führt von der Branche 20 ein nicht weiter veranschaulichter Leiter zu dem Gerät. Der Leiter 44 kann bspw. ein mit einer Isolierung 46 versehener Runddraht oder dergleichen sein. Er ist etwa zentral an einer zwischen der Elektrode 33 und dem Grundkörper 23 liegenden Stelle an die Elektrode 33 angeschlossen. Insbesondere im Scharnierbereich 30 ist der Leiter 44 durch eine seitliche Öffnung 47 des Grundkörpers 23 an dessen Außenseite geführt und verläuft dort in einer Längsausnehmung 49 bis zu dem Ansatz 24. Dort tritt der Leiter 44 in den Schaft 11 ein. Der Leiter 44 kann insbesondere ein isolierter Silberdraht sein.

Das insoweit beschriebene Instrument 10 arbeitet wie folgt:
zum Schließen und Trennen eines Gefäßes wird dieses zwischen den Branchen 19, 20 gefasst. Durch Betätigung des Griffteils 15 werden die Branchen 19, 20 aufeinander zu bewegt, so dass das Gefäß abgeklemmt wird und die Gefäßwände aneinander gedrückt werden. Die Gefäßwände werden dabei insbesondere zwischen der Elektrodenfläche 40 und der Elektrode 33 zusammengedrückt. Sie werden zusätzlich zwischen den Vorsprüngen 38, 39 und der Elektrodenfläche 40 oder den Flanken 42, 43 gefasst. Im Idealfall sitzt der Vorsprung 41 auf dem als Widerlager dienenden Bereich 34 des Grundkörpers 23 und verhindert somit ein Abquetschen des Gefäßes.

Durch Anlegen eines Stroms oder Spannung zwischen der Branche 20 und der Elektrode 33, vorzugsweise einer HF-Spannung wird ein Koagulationsprozess eingeleitet, in dessen Verlauf Zellen der Gefäßwände geöffnet, Eiweiß freigesetzt und denaturiert wird. Es kommt zum Verkleben der zwischen der Branche 20 und der Elektrode 33 gefassten aneinander gedrückten Gefäßwände. Diese können dabei an Volumen verlieren. Unabhängig vom Schwinden des Volumens der Gefäßwände bleiben jedoch die zwischen den Vorsprüngen 38, 39 und der Branche 20 gefassten Partien der Gefäße, zumindest weitgehend, in natürlichem Zustand und unterliegen somit kaum einer Schwindung. Sie bleiben sicher gefasst, wodurch ein seitliches Verrutschen des Gefäßes verhindert wird, dies hat insbesondere beim Schließen relativ kleiner Gefäße Bedeutung.

Ist hingegen ein Geweberest zwischen die Nase 41 und den als Widerlager dienenden Bereich 34 geraten, so dass die Spaltweite A etwas größer als eigentlich vorgesehen ist, wirkt wiederum der enge Spalt B als Sicherung gegen seitliches Verrutschen des Gefäßes. Außerdem kann der Abstand zwischen der Nase 41 und dem vorderen Ende 48 der Elektrode 33 so gering sein, dass hier vorhandenes Gewebe koaguliert wird und somit schwindet. Auch dies kommt wiederum dem sicheren Fassen des Gewebes zwischen den Branchen 19, 20 zugute.

Ist der Koagulationsprozess ausreichend weit fortgeschritten kann der Chirurg z.B. durch Betätigung des Knopfs 16 das Messer 21 aktivieren. Es wird nun von dem Führungsschlitz 25 und dem Längsschlitz 31 mittig geführt zu dem freien Ende der Branchen 19, 20 hin geschoben. Das Messer 21 läuft dabei mehr oder weniger genau innerhalb einer Mittelebene M, die in Figur 4 strichpunktiert angedeutet ist, damit wird sichergestellt, dass zu beiden Seiten des Messers 21 gleich lange Bereiche geschlossener Gefäßenden entstehen. Die Gefahr eines außermittigen Schnitts und somit die Gefahr unkontrollierter Blutungen ist dadurch minimiert.

Ein vorzugsweise zur minimalinvasiven Chirurgie geeignetes z.B. durch einen Trokar in einen Körper einführbares Instrument 10 weist zum Abklemmen von Gefäßen zwei Branchen 19, 20 und ein mittig geführtes Messer 21 auf. Durch die mittige Führung des Messers in zumindest einer der Branchen 19, 20 werden Nachteile vermieden, wie sie sonst infolge außermittiger Trennung des koagulierten Gewebes entstehen könnten. Außerdem ergibt sich durch die saubere mittige Führung des Messers 21 ein verminderter Verschleiß desselben. Die Erfindung ermöglicht somit eine bessere Schnittqualität und eine längere Standzeit des Messers 21.

Eine der Branchen weist einen vorzugsweise keramischen Grundkörper 23 auf, an dessen oberen Rändern nach innen ragende Vorsprünge 38, 39 ausgebildet sind, die die Form eines Hinterschnittes aufweisen. Diese elektrisch isolierenden Vorsprünge erstrecken sich in Richtung zu der Mittelebene M hin und erbringen zwei Verbesserungen: Zum einen wird die Montage der Elektrode 33 erheblich erleichtert. Zum anderen werden zwei Gewebespannstellen geschaffen. Die Vorsprünge können so ausgebildet sein, dass bei geschlossenen Branchen 20, 19 der Abstand A zwischen der durch die obere Branche 20 gebildeten Elektrode und der Elektrode 33 an der Stelle, an der der Abstand A seine größte Weite aufweist, und die beiden Abstände B zwischen den Vorsprüngen 38, 39 und der Branche 20 gleich, nahezu gleich oder geringfügig kleiner als der Abstand A ist. Dies hat den Vorteil, dass während des Koagulationsprozesses, bei dem das Gewebevolumen im Koagulationsbereich abnimmt, das Gewebe trotzdem sicher gehalten wird. Der Abstand A verjüngt sich vorzugsweise ausgehend von den Vorsprüngen 38, 39 bis zu dem Führungsschlitz 31 hin. Bei dem vorliegenden Instrument wird die Koagulationsbreite durch die isolierenden Vorsprünge 38, 39 bestimmt und ist somit sicher reproduzierbar; dies verbessert die Koagulationsqualität erheblich.

### Bezugszeichenliste:

- 10: Instrument
- 11: Schaft
- 12: Gehäuse
- 13: Handgriff
- 14: Betätigungselemente
- 15: Griffteil
- 16: Betätigungsknopf oder -Hebel
- 17: Kraftübertragungsmittel
- 18: Anwendungsteil, Kopf
- 19: erste Branche
- 20: zweite Branche
- 21: Messer
- 22: Pfeil
- 23: Grundkörper
- 24: Ansatz
- 25: Führungsschlitz
- 26, 27: Schlitzflanken
- S: Spiel zwischen 25 und 21
- D: Dicke des Messers 21
- DK: Dicke der Klinge 28
- 28: Klinge
- 28a: Gleitkante
- 28b: Spitze
- 28c: Schneidkante
- 29: Führungsleiste
- 30: Scharnierbereich
- 31: Längsschlitz
- S1: Spiel zwischen 31 und 21
- 32: Ausnehmung
- 33: Elektrode
- 34: endständiger Bereich des Grundkörpers 23
- 35, 36: obere Ränder
- 37: Haltemittel
- 38, 39: Rastvorsprünge
- 40: Elektrodenfläche
- 41: Vorsprung
- A: Weite des Abstandsspalts zwischen 33 und 40
- B: Abstand zwischen 38, 39 und 40
- 42, 43: Flanke der Branche 20
- 44: Leiter
- 45: Zuleitung
- 46: Isolierung
- 47: seitliche Öffnung des Grundkörpers 23
- 48: vorderes Ende von 33
- M: Mittelebene
- 49: Längsausnehmung
- 52, 53: Schlitzflanken von 31

## Patentansprüche

1. Instrument (10) zur Gefäßfusion und Trennung
mit einer ersten Branche (19), die einen Grundkörper (23) mit einer rinnenartigen Ausnehmung (32) und mit einem Führungsschlitz (25) aufweist, wobei an dem Grundkörper (23) Haltemittel (37, 38, 39) zur ortsfesten Lagerung einer gekrümmt ausgebildeten Elektrode (33) ausgebildet sind,
mit einer zweiten Brache (20), die an dem Grundkörper (23) auf die Elektrode (33) zu und von dieser weg beweglich gelagert ist, wobei die zweite Branche (20) einen Längsschlitz (31) aufweist,
mit einem Messer (21), das in dem Führungsschlitz (25) des Grundkörpers (23) längsbeweglich angeordnet und in den Längsschlitz (31) hineinragend oder in diesen einfahrbar angeordnet ist,
**dadurch gekennzeichnet,**
**dass** der Grundkörper (23) elektrisch isolierend ausgebildet ist und
**dass** die Haltemittel (37, 38, 39) durch elektrisch isolierende Vorsprünge gebildet sind, die nach innen in Richtung auf die Ausnehmung (32) zu und über den oberen Rand der Elektrode ragen und somit den oberen Rand der Elektrode (33) überdecken.

2. Instrument nach Anspruch 1, **dadurch gekennzeichnet, dass** das Messer (21) eine Führungsleiste (29) und eine Klinge (28) umfasst, die fest miteinander verbunden sind.

3. Instrument nach Anspruch 2, **dadurch gekennzeichnet, dass** das seitliche Spiel (S) des Messers (21) in dem Führungsschlitz (25) geringer ist als das Fünffache der Dicke (D) der Klinge (28).

4. Instrument nach Anspruch 1, **dadurch gekennzeichnet, dass** das Messer (28) eine gerade, der Elektrode (33) zugewandte und an dieser anliegende, längsverlaufende Gleitkante (28a) aufweist.

5. Instrument nach Anspruch 1, **dadurch gekennzeichnet, dass** das Messer (21) eine stirnseitige Schneidkante (28c) aufweist, die in einem Winkel zu der Bewegungsrichtung des Messers (21) orientiert ist.

6. Instrument nach Anspruch 1, **dadurch gekennzeichnet, dass** das Messer (21) an der von der Elektrode (33) abgewandten Seite eine Führungsleiste (29) aufweist, die in dem Führungsschlitz (25) des Grundkörpers (23) längsbeweglich angeordnet und in den Längsschlitz (31) der zweiten Branche (20) hineinragend oder in diesen einfahrbar angeordnet ist und die das seitliche Spiel (S) des Messers (21) in dem Grundkörper (23) und in der zweiten Branche (20) festlegt.

7. Instrument nach Anspruch 1, **dadurch gekennzeichnet, dass** die zweite Branche (20) durch einen massiven Metallkörper gebildet ist.

8. Instrument nach Anspruch 1, **dadurch gekennzeichnet, dass** die zweite Branche (20) an ihrer der Elektrode (33) der ersten Branche (19) zugewandten Seite eine der Form der Elektrode (33) folgende Form aufweist.

9. Instrument nach Anspruch 1, **dadurch gekennzeichnet, dass** bei geschlossenen Branchen 19 und 20 des Instruments (10) zwischen der Elektrode (33) und der zweiten Branche (20) ein Abstandsspalt mit einer Weite (A) festgelegt ist.

10. Instrument nach Anspruch 1, **dadurch gekennzeichnet, dass** die Haltemittel (37) an beiden einander gegenüberliegenden Rändern (35, 36) der Ausnehmung (32) ausgebildet sind.

11. Instrument nach Anspruch 9 und 10, **dadurch gekennzeichnet, dass** die Haltemittel (37) bei geschlossenen Branchen (19, 20) des Instruments (10) mit der zweiten Branche (20) einen Abstand (B) festlegen, der gleich oder geringer ist, als die Weite (A) des Abstandsspalts.

12. Instrument nach Anspruch 1, **dadurch gekennzeichnet, dass** die Elektrode (33) über eine wenigstens abschnittsweise an der Außenseite des Grundkörpers (23) angeordnete elektrische Zuleitung (44) mit einer elektrischen Versorgungsquelle verbunden ist.

13. Instrument nach Anspruch 1, **dadurch gekennzeichnet, dass** der Grundkörper (23) mit einem länglichen Schaft (11) verbunden ist, der sich von einem Gehäuse (12) weg erstreckt, wobei der Schaft (11) Kraftübertragungsmittel (17) zur gezielten Bewegung der zweiten Branche (20) und/oder des Messers (21) aufweist.

14. Instrument nach Anspruch 1, **dadurch gekennzeichnet, dass** an der ersten und/oder der zweiten Branche (19, 20) ein Abstandshaltemittel vorgesehen ist.

15. Instrument nach Anspruch 14, **dadurch gekennzeichnet, dass** das Abstandshaltemittel ein metallischer, elektrisch leitender oder ein elektrisch isolierender Vorsprung (41) ist.

## Claims

1. Instrument (10) for fusing and cutting vessels
with a first arm (19), which has a base body (23) with a groove-like recess (32) and with a guide slot (25), wherein holding elements (37, 38, 39) are configured on the base body (23) for the fixed-position mounting of a curved electrode (33),
with a second arm (20), which is mounted on the base body (23) to be movable towards and away from the electrode (33), wherein the second arm (20) has a longitudinal slot (31),
with a knife (21), which is arranged to be longitudinally movable in the guide slot (25) of the base body (23) and is arranged to project into the longitudinal slot (31) or to be insertable into this,
**characterised in that**
the base body (23) is electrically isolated and
that the holding elements (37, 38, 39) are formed by electrically isolating projections, which project inwards in the direction of the recess (32) and beyond the upper edge of the electrode, and thus cover the upper edge of the electrode (33).

2. Instrument according to claim 1, **characterised in that** the knife (21) comprises a guide strip (29) and a blade (28), which are fixedly connected to one another.

3. Instrument according to claim 2, **characterised in that** the lateral play (S) of the knife (21) in the guide slot (25) is less than five-times the thickness (D) of the blade (28).

4. Instrument according to claim 1, **characterised in that** the knife (28) has a straight, longitudinally running sliding edge (28a) facing the electrode (33) and abutting this.

5. Instrument according to claim 1, **characterised in that** the knife (21) has a cutting edge (28c) on the front side, which is oriented at an angle to the direction of movement of the knife (21).

6. Instrument according to claim 1, **characterised in that** on the side remote from the electrode (33) the knife (21) has a guide strip (29), which is arranged to be longitudinally movable in the guide slot (25) of the base body (23) and is arranged to project into the longitudinal slot (31) of the second arm (20) or to be insertable into this and which fixes the lateral play (S) of the knife (21) in the base body (23) and in the second arm (20).

7. Instrument according to claim 1, **characterised in that** the second arm (20) is formed by a solid metal body.

8. Instrument according to claim 1, **characterised in that** on its side facing the electrode (33) of the first arm (19) the second arm (20) has a shape that follows the shape of the electrode (33).

9. Instrument according to claim 1, **characterised in that** when the arms (19 and 20) of the instrument (10) are closed a distance gap with a width (A) is defined between the electrode (33) and the second arm (20).

10. Instrument according to claim 1, **characterised in that** the holding elements (37) are configured on the two opposing edges (35, 36) of the recess (32).

11. Instrument according to claim 9 and 10, **characterised in that** when the two arms (19 and 20) of the instrument (10) are closed the holding elements (37) define a space (A) that is equal to or smaller than the width (A) of the distance gap.

12. Instrument according to claim 1, **characterised in that** the electrode (33) is connected to an electrical supply source by means of an electrical supply line (44) arranged at least in sections on the outer surface of the base body (23).

13. Instrument according to claim 1, **characterised in that** the base body (23) is connected to an elongated shaft (11), which extends away from a housing (12), wherein the shaft (11) has force transfer elements (17) for selective movement of the second arm (20) and/or the knife (21).

14. Instrument according to claim 1, **characterised in that** a spacer element is provided on the first and/or the second arm (19, 20).

15. Instrument according to claim 14, **characterised in that** the spacer element is a metallic electrically conductive or an electrically isolating projection (41).

## Revendications

1. Instrument (10) pour la fusion de vaisseaux et le sectionnement,
comprenant un premier mors (19) qui présente un corps de base (23) comportant un évidement (32) en forme de rainure et une fente de guidage (25), des moyens de fixation (37, 38, 39) étant réalisés sur le corps de base (23) en vue du montage fixe d'une électrode (33) réalisée sous une forme incurvée,
comprenant un deuxième mors (20) qui est monté sur le corps de base (23) avec possibilité de déplacement pour s'approcher de l'électrode (33) et s'en éloigner, le deuxième mors (20) présentant une fente longitudinale (31),
comprenant un couteau (21) qui est disposé avec possibilité de déplacement longitudinal dans la fente de guidage (25) du corps de base (23) et est disposé de façon à s'étendre dans la fente longitudinale (31) ou à pouvoir être engagé dans celle-ci,
**caractérisé en ce que**
le corps de base (23) est réalisé de façon électriquement isolante, et
**en ce que** les moyens de fixation (37, 38, 39) sont constitués de saillies électriquement isolantes qui avancent vers l'intérieur, en direction de l'évidement (32), et au-dessus du bord supérieur de l'électrode et recouvrent ainsi le bord supérieur de l'électrode (33).

2. Instrument selon la revendication 1, **caractérisé en ce que** le couteau (21) comprend une baguette de guidage (29) et une lame (28) qui sont liées solidement l'une à l'autre.

3. Instrument selon la revendication 2, **caractérisé en ce que** le jeu latéral (S) du couteau (21) dans la fente de guidage (25) est inférieur au quintuple de l'épaisseur (D) de la lame (28).

4. Instrument selon la revendication 1, **caractérisé en ce que** le couteau (28) présente une arête de glissement (28a) rectiligne, s'étendant dans le sens longitudinal, qui est tournée vers l'électrode (33) et est appliquée contre celle-ci.

5. Instrument selon la revendication 1, **caractérisé en ce que** le couteau (21) présente une arête coupante (28c) côté frontal, qui est orientée en formant un angle avec le sens de déplacement du couteau (21).

6. Instrument selon la revendication 1, **caractérisé en ce que** le couteau (21), sur le côté éloigné de l'électrode (33), présente une baguette de guidage (29) qui est disposée avec possibilité de déplacement longitudinal dans la fente de guidage (25) du corps de base (23) et est disposée de façon à s'étendre dans la fente longitudinale (31) du deuxième mors (20) ou à pouvoir être engagée dans celle-ci, et qui détermine le jeu latéral (S) du couteau (21) dans le corps de base (23) et dans le deuxième mors (20).

7. Instrument selon la revendication 1, **caractérisé en ce que** le deuxième mors (20) est constitué d'un corps métallique plein.

8. Instrument selon la revendication 1, **caractérisé en ce que** le deuxième mors (20), sur son côté tourné vers l'électrode (33) du premier mors (19), présente une forme qui suit la forme de l'électrode (33).

9. Instrument selon la revendication 1, **caractérisé en ce que**, lorsque les mors (19) et (20) de l'instrument (10) sont fermés, une fente d'espacement d'une largeur (A) est définie entre l'électrode (33) et le deuxième mors (20).

10. Instrument selon la revendication 1, **caractérisé en ce que** les moyens de fixation (37) sont réalisés sur les deux bords (35, 36) opposés de l'évidement (32).

11. Instrument selon la revendication 9 et 10, **caractérisé en ce que**, lorsque les mors (19, 20) de l'instrument (10) sont fermés, les moyens de fixation (37) définissent avec le deuxième mors (20) une distance (B) qui est égale ou inférieure à la largeur (A) de la fente d'espacement.

12. Instrument selon la revendication 1, **caractérisé en ce que** l'électrode (33) est reliée à une source d'alimentation électrique par un câble d'arrivée (44) électrique disposé au moins en partie sur la face extérieure du corps de base (23).

13. Instrument selon la revendication 1, **caractérisé en ce que** le corps de base (23) est relié à une tige (11) allongée qui s'étend à partir d'un boîtier (12), la tige (11) présentant des moyens de transmission de force (17) en vue du mouvement précis du deuxième mors (20) et/ou du couteau (21).

14. Instrument selon la revendication 1, **caractérisé en ce qu'**un moyen d'esplacement est prévu sur le premier et/ou le deuxième mors (19, 20).

15. Instrument selon la revendication 14, **caractérisé en ce que** le moyen d'espacement est une saillie (41) métallique, électriquement conductrice ou électriquement isolante.
